(19) Europäisches Patentamt
European Patent Office
Office européen des brevets

(11) EP 4 265 176 A1

(12) EUROPEAN PATENT APPLICATION
published in accordance with Art. 153(4) EPC

(43) Date of publication:
25.10.2023 Bulletin 2023/43

(51) International Patent Classification (IPC):
A61B 5/01 (2006.01)     A61B 5/00 (2006.01)

(21) Application number: 21894857.8

(22) Date of filing: 23.09.2021

(52) Cooperative Patent Classification (CPC):
A61B 5/00; A61B 5/01

(86) International application number:
PCT/KR2021/012978

(87) International publication number:
WO 2022/108084 (27.05.2022 Gazette 2022/21)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 18.11.2020 KR 20200154217

(71) Applicant: Osong Medical Innovation Foundation
Cheongju-si, Chungcheongbuk-do 28160 (KR)

(72) Inventors:
• LEE, Seung Rag
  Sejong 30064 (KR)
• HONG, Sung Jun
  Sejong 30153 (KR)
• PARK, Byung Jun
  Sejong 30064 (KR)
• KIM, Byung Yeun
  Cheongju-si Chungcheongbuk-do 28165 (KR)
• LEE, Min Suk
  Cheongju-si Chungcheongbuk-do 28165 (KR)

(74) Representative: Lewis Silkin LLP
Arbor
255 Blackfriars Road
London SE1 9AX (GB)

(54) TEMPERATURE SENSOR, WEARABLE DEVICE COMPRISING TEMPERATURE SENSOR, CORE TEMPERATURE MEASUREMENT METHOD USING TEMPERATURE SENSOR

(57) In a temperature sensor, a wearable device comprising the temperature sensor, and a core temperature measurement method using the temperature sensor, the temperature sensor includes a sensing unit and a substrate. The sensing unit includes a base part, a measuring part mounted on the base part and configured to measure a temperature of a subject body by making contact with the subject body, and a cover part configured to cover the measuring part. The sensing unit is mounted on the substrate. A plurality of slits is formed around a first area of the substrate, and the sensing unit is mounted at the first area of the substrate.

FIG. 1A

**Description**

BACKGROUND OF THE INVENTION

TECHNICAL FIELD

Exemplary embodiments of the present invention relate to a temperature sensor, a wearable device comprising the temperature sensor, and a core temperature measurement method using the temperature sensor. More particularly, exemplary embodiments of the present invention relate to a temperature sensor, a wearable device comprising the temperature sensor, and a core temperature measurement method using the temperature sensor capable of measuring a core temperature of a body considering an ambient temperature efficiently. Here, the wearable device is manufactured as a band type or a patch type for easily being attached to the body.

DISCUSSION OF THE RELATED ART

Recently, various types of products related to health care are developed in a situation where infectious diseases spread and the importance of managing personal health is emphasized.
As wearable health care related products are developed, utilization is increasing, such as obtaining information on bio-signals of various users, for example, electrocardiogram, oxygen saturation, temperature, pulse, blood pressure, activity level and so on, analyzing and monitoring the information, performing patient-specific health management monitoring, or using the information as a database in providing medical services.
In these wearable health care products, the most important thing is to measure the user's bio-signals accurately. In particular, the most important of the user's bio-signals is the user's body temperature, particularly information on the core body temperature.
Regarding the measurement of the core body temperature, Korean laid-open patent No. 10-2020-0061579 or No. 10-2020-0061580 discloses the technology for the core body temperature measuring device that enters the user's ear and measures the core body temperature. However, in the prior arts, the device has a complicated structure, is not easy to manufacture, and is not easy to implement the device as a wearable device, so that there are problems such as deterioration in usability of users.
As explained above, the related prior arts are Korean laid-open patents No. 10-2020-0061579 and No. 10-2020-0061580.

SUMMARY

Exemplary embodiments of the present invention provide a temperature sensor capable of measuring a core temperature of a body considering an ambient temperature efficiently and capable of being applied to a wearable device which is manufactured as a band type or a patch type for easily being attached to the body.
Exemplary embodiments of the present invention also provide a wearable device comprising the temperature sensor.
Exemplary embodiments of the present invention further also provide a core temperature measurement method using the temperature sensor.
According to one aspect of the present invention, the temperature sensor includes a sensing unit and a substrate. The sensing unit includes a base part, a measuring part mounted on the base part and configured to measure a temperature of a subject body by making contact with the subject body, and a cover part configured to cover the measuring part. The sensing unit is mounted on the substrate. A plurality of slits is formed around a first area of the substrate, and the sensing unit is mounted at the first area of the substrate.
In an exemplary embodiment, the sensing unit may further include a terminal part extending from the base part toward an opposite to the measuring part, and passing through the substrate to be electrically connected to the substrate.
In an exemplary embodiment, the cover part may cover an entire of the measuring part except for a portion of the measuring part heading for the subject body, and the portion of the measuring part exposed toward the subject body may make contact with the subject body for measuring the temperature of the subject body.
In an exemplary embodiment, the cover part may include a measuring sensor configured to measure an ambient temperature.
In an exemplary embodiment, the slits may be spaced apart from the first area by a predetermined distance, and the slits may be spaced apart from each other by a predetermined distance.
In an exemplary embodiment, the distance between the slits adjacent to each other, may be smaller than a length of each of the slits.
According to another aspect of the present invention, the wearable device includes the temperature sensor, a vital sign measuring part, a display part and a case. The vital sign measuring part is configured to measure vital signs of the subject body except for the temperature of the subject body. The display part is configured to display results of the temperature sensor and the vital sign measuring part. The case receives the temperature sensor, the vital sign measuring part and the display part.

In an exemplary embodiment, the measuring part of the temperature sensor may be exposed to an outside of the case to make contact with the subject body.

According to still another aspect of the present invention, the wearable device includes the temperature sensor, a vital sign measuring module, a communication module and a connecting part. The vital sign measuring module includes a vital sign measuring part and the vital sign measuring part is configured to measure vital signs of the subject body except for the temperature of the subject body. The communication module is disposed to an opposite to the vital sign measuring module and is configured to communicate with outside. The connecting part is configured to connect the vital sign measuring module with the communication module. The temperature sensor is disposed inside of the vital sign measuring module or the connecting part.

In an exemplary embodiment, the connecting part may be a flexible structure, and the measuring part of the temperature sensor may be exposed to an outside of the connecting part or an outside of the vital sign measuring module to make contact with the subject body.

In an exemplary embodiment, the wearable device may further include a first electrode attached to a lower portion of the vital sign measuring module to make contact with the subject body, and a second electrode attached to a lower portion of the communication part to make contact with the subject body.

According to still another aspect of the present invention, in a core temperature measurement method using the temperature sensor, an ambient temperature is obtained. A core body temperature is obtained using the temperature sensor. The core body temperature is corrected based on the ambient temperature.

In an exemplary embodiment, in the correcting the core body temperature, the ambient temperature may be compared to the core body temperature, and then when the ambient temperature satisfies a first temperature condition, the core body temperature may not be corrected and is considered to be a core temperature of the subject body, and when the ambient temperature satisfies a second temperature condition, the core body temperature may be corrected to obtain a core temperature of the subject body.

In an exemplary embodiment, in the first temperature condition, the ambient temperature may be in a range between 24°C and 26°C, and in the second temperature condition, the ambient temperature may be less than 24°C or over 26°C.

In an exemplary embodiment, in the correcting the core body temperature, the core body temperature may be corrected using Equation (1) below,

$$T_C = T_O + T_A \times C_W \qquad\qquad \text{Equation (1)}$$

Here, $T_c$ may be a corrected core body temperature, $T_o$ may be a measured core body temperature, $T_A$ may be a measured ambient temperature, and $C_w$ may be a correcting coefficient.

According to some exemplary embodiments of the present invention, the measuring part is mounted on the substrate to be operated, but heat generated from various elements may be provided to the measuring part through the substrate, so that the measured temperature of the subject body may be inaccurate.

Thus, in the present example embodiments, the plurality of slits is formed in the substrate and thus a first area in which the sensing unit is formed is isolated. Thus, the heat transfer through the substrate may be minimized and the measured temperature of the subject body may be accurate.

Here, a portion of the measuring part heading for the subject body is merely exposed and other entire of the measuring part is blocked by the cover, and thus measured temperature of the subject body may be accurate.

In addition, the temperature sensor may be applied to the band type or the patch type wearable device. When applied to the patch type wearable device, the temperature sensor may be mounted on the connecting part having the flexible structure, to increase the adhesiveness with the subject body.

Further, in the wearable device, various kinds of vital signs in addition to the body temperature may be obtained, and the vital signs may be obtained using the electrode making contact with the subject body.

Here, in measuring the core body temperature, to increase the accuracy of the measurement, the core body temperature is corrected using the ambient temperature.

BRIEF DESCRIPTION OF THE DRAWINGS

FIG. 1A and FIG. 1B are a cross-sectional view and a plan view respectively illustrating a temperature sensor according to an example embodiment of the present invention;

FIG. 2A, FIG. 2B and FIG. 2C are a perspective view, a bottom view and a cross-sectional view respectively illustrating a band type wearable device having the temperature sensor of FIG. 1A;

FIG. 3A and FIG. 3B are a perspective view and a cross-sectional view respectively illustrating a patch type wearable device having the temperature sensor of FIG. 1A; and

FIG. 4 is a flow chart showing a core temperature measurement method using the temperature sensor of FIG. 1A, and FIG. 5 is a flow chart showing the flow chart of FIG. 4 in detail.

<reference numerals>

| 1 : | temperature sensor | 10 : | sensing unit |
|---|---|---|---|
| 20 : | substrate | 21 : | slit |
| 22 : | gap | 30 : | band type wearable device |
| 33 : | vital sign measuring part | 40 : | patch type wearable device |
| 41 : | vital sign measuring module | 43 : | communication module |
| 44 : | connecting part | 45, 46 : | electrode |

DETAILED DESCRIPTION OF THE INVENTION

The present invention is described more fully hereinafter with reference to the accompanying drawings, in which exemplary embodiments of the present invention are shown. The present invention may, however, be embodied in many different forms and should not be construed as limited to the exemplary embodiments set forth herein. Rather, these exemplary embodiments are provided so that this disclosure will be thorough and complete, and will fully convey the scope of the present invention to those skilled in the art.

The terminology used herein is for the purpose of describing particular exemplary embodiments only and is not intended to be limiting of the present invention. As used herein, the singular forms "a," "an" and "the" are intended to include the plural forms as well, unless the context clearly indicates otherwise.

It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, the present invention will be explained in detail with reference to the accompanying drawings.

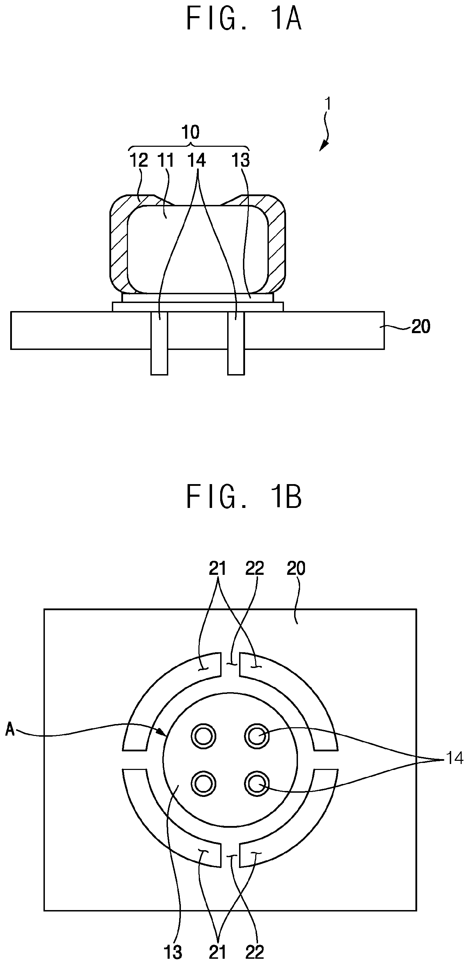

FIG. 1A and FIG. 1B are a cross-sectional view and a plan view respectively illustrating a temperature sensor according to an example embodiment of the present invention.

Referring to FIG. 1A and FIG. 1B, the temperature sensor 1 according to the present example embodiment includes a sensing unit 10 and a substrate 20.

The sensing unit 10 includes a measuring part 11, a cover part 12, a base part 13 and a terminal part 14. The sensing unit 10 is mounted on the substrate 20.

Although not shown in the figure, various kinds of elements may be mounted on the substrate 20, and electric wires between the sensing unit 10 and the elements may be formed on the substrate 20.

Here, the substrate 20 may be a printed circuit board (PCB) or a flexible printed circuit board (FPCB).

The base part 13 is disposed on the substrate 20, and the terminal part 14 extends from a side of the base 13, for example from a lower side of the base 13.

The terminal part 14 passes through an opening formed through the substrate 20, and the terminal part 14 is electrically connected to the substrate 20.

Here, the number of the terminal parts 14 connected to the substrate 20 is not limited, and for example, four terminals parts 14 may be formed as illustrated in FIG. 1B.

The measuring part 11 is disposed on the base part 13 along a direction opposite to the extending direction of the terminal part 14, for example an upper direction from the base part 13.

The measuring part 11 makes direct contact with a subject body, and measures a temperature of the subject body. Here, the measuring part 11 is sufficient to measure the temperature of the subject body, and the type of the measuring part 11 not limited.

The temperature measured by the measuring part 11 is a core body temperature not a surface temperature of the body, and thus the measuring part 11 should be the sensor capable of measuring the core body temperature of the subject body.

Here, as illustrated in FIG. 1A, an upper surface of the measuring part 11 is merely exposed, and other surfaces of the measuring part 11 is covered by the cover part 12.

The cover part 12 covers the measuring part 11 in the form of capsules, and thus the exposed upper surface of the measuring part 11 only makes direct contact with the subject body.

Here, the cover part 12 may include an insulating material.

Accordingly, the measuring part 11 is entirely covered by the cover part 12 except for the portion making contact with the subject body, and thus the heat transferred from outside may be prevented from being transferred to the measuring part 11 and the temperature of the subject boy may be measured more accurately.

The cover part 12 includes a measuring sensor (not shown), and an ambient temperature may be obtained by the measuring sensor of the cover part 12. Here, the ambient temperature may be obtained in addition to the core body temperature.

In the present example embodiment, the plurality of slits 21 is formed in a first area A in which the sensing unit 10 having the base part 13 is mounted.

The slits 21 are the openings which are formed by passing through the substrate 20. As the slits 21 are formed via passing through the substrate 20, the sensing unit 10 may be isolated in the substrate 20.

Here, when the base part 13 has a circular shape, the plurality of the slits is formed around the first area A having the circular shape, and each of the slits is spaced apart from the first area A by a predetermined distance.

In addition, the slits 21 are space apart from each other by a predetermined distance. For example, the slits 21 adjacent to each other may form a gap 22.

Thus, as illustrated in FIG. 1B, when four slits 21 are formed, each of the slits 21 may be a circumferential shape slit forming about 90°. In addition, the slits 21 form the circular shape slit around the first area A, and the gap 22 is formed between the slits adjacent to each other. Thus, the gap 22 is formed at every 90°.

The sensing unit 10 should be electrically connected to the elements formed on the substrate 20, to receive a power from the substrate 20 or to be controlled by an electrical signal.

Thus, in the present example embodiment, the electric connection for operating the sensing unit 10 is maintained via the gap 22, and all areas around the sensing unit 10 except for the minimum configuration for the electrical connection mentioned above are formed with the opening slits 21.

Various kinds of elements may be mounted on the substrate 20, and the heat generated from the elements may be transferred, so that the heat from the substrate 20 may be also transferred to the sensing unit 10. Thus, the results measured by the sensing unit 10 may be changed by the heat from the substrate 20 in addition to the heat from the subject body.

Thus, the slits 21 formed around the sensing unit 10 may prevent the heat from the substrate 20, and thus the results measured by the sensing unit 10 depends on only the heat from the subject body and the measured temperature may be accurate.

Thus, the accuracy of the measured results by the temperature sensor 1 may be more enhanced.

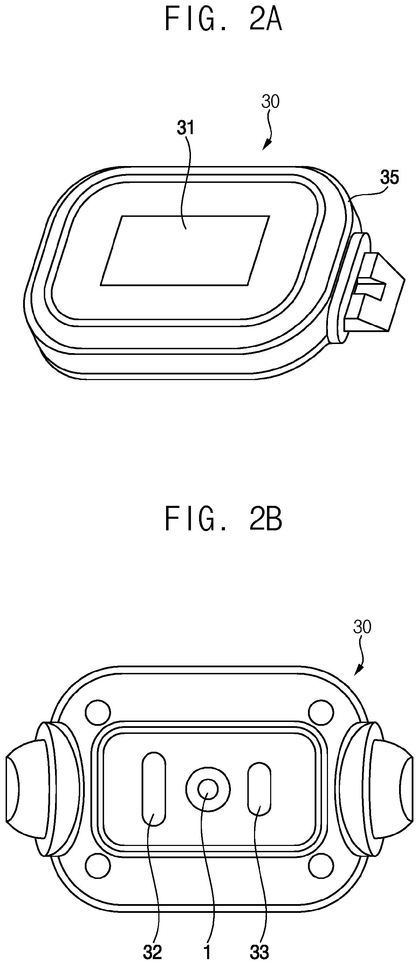

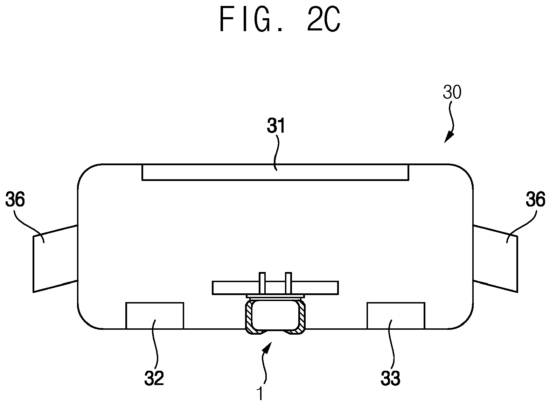

FIG. 2A, FIG. 2B and FIG. 2C are a perspective view, a bottom view and a cross-sectional view respectively illustrating a band type wearable device having the temperature sensor of FIG. 1A.

Referring to FIG. 2A, FIG. 2B and FIG. 2C, the band type wearable device 30 (hereinafter, the wearable device) according to the present example embodiment includes a case 35, a band part 36, a display part 31, a power supply 32 and a vital sign measuring part 33, in addition to the temperature sensor 1 explained above referring to FIG. 1A and FIG. 1B.

The temperature sensor 1 is substantially same as explained above referring to FIG. 1A and FIG. 1B, and any repetitive explanation will be omitted.

The case 35, as illustrated in the figure, has a front side and a rear side, and the shape of the case 35 is not limited. The display part 31 is formed at the front side, and the temperature sensor 1, the power supply 32 and the vital sign measuring part 33 are formed at the rear side.

Here, the rear side at which the temperature sensor 1, the power supply 32 and the vital sign measuring part 33 is the surface directly making contact with the subject body such as a wrist. Thus, the front side heads for an outside, and the measured results by the temperature sensor 1 and the vital sign measuring part 33 are displayed by the display part 31 of the front side.

The band part 36 is connected to both sides of the case 35, and fixes the case 35 to the wrist of the subject body.

Accordingly, the wearable device 30 is manufactured with the band type, and is fixed to the wrist of the subject body.

The power supply 32 provides a power source to the temperature sensor 1, the vital sign measuring part 33 and the display part 31.

Here, the temperature sensor 1, as illustrated in the figure, is fixed to the case 35, with an exposed portion of the measuring part 11 heading for a lower portion of the wearable device 30. Thus, the body temperature of the subject body may be measured by the exposed portion of the measuring part 11.

The vital sign measuring part 33 measures other vital information of the subject body except for the temperature of the subject body which is measured by the temperature sensor 1.

For example, the vital sign measuring part 33 may measure oxygen saturation, pulse, blood pressure, electrocardiogram and so on, and here the kind or the structure of the vital sign measuring part 33 is not limited.

Accordingly, the temperature sensor 1 is configured at the band type wearable device 30, and the exposed portion of the measuring part 11 of the temperature sensor 1 is disposed to head for the subject body. Then, the temperature of the subject body may be measured.

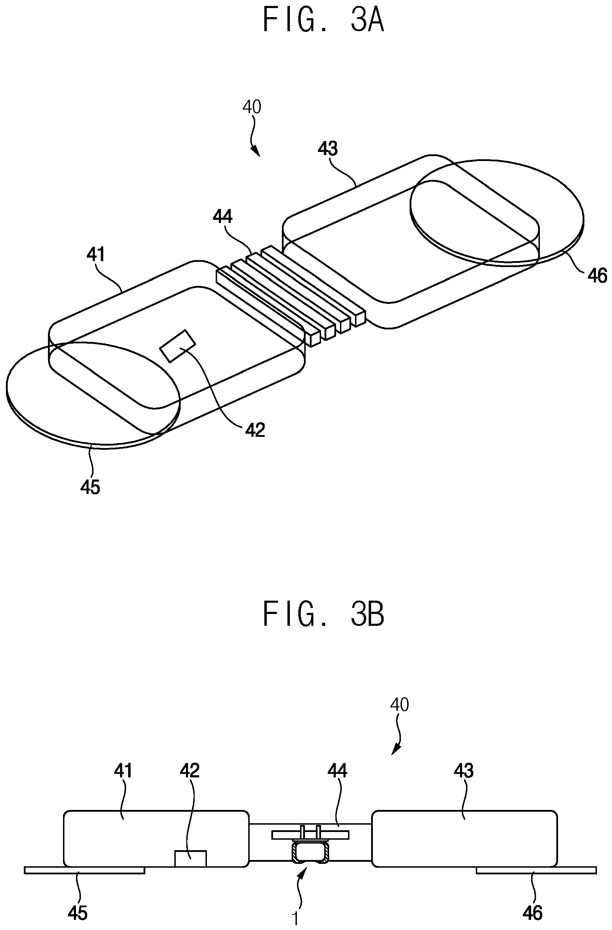

FIG. 3A and FIG. 3B are a perspective view and a cross-sectional view respectively illustrating a patch type wearable device having the temperature sensor of FIG. 1A.

Referring to FIG. 3A and FIG. 3B, the patch type wearable device 40 (hereinafter, the wearable device) according to the present example embodiment includes a vital sign measuring module 41, a communication module 43, a connecting part 44, a first electrode 45 and a second electrode 46, in addition to the temperature sensor 1 as explained above referring to FIG. 1A and FIG. 1B.

Here, the temperature sensor 1 is substantially same as explained above, and thus any repetitive explanation will be omitted.

The vital sign measuring module 41, as illustrated in the figure, may have a rectangular block shape with a flat shape, and a vital sign measuring part 42 may be disposed inside of the vital sign measuring module 41.

Here, the vital sign measuring part 42 is substantially same as the vital sign measuring part 33 explained above referring to FIG. 2C.

In addition, although not shown in the figure, other elements capable of measuring other vital signs may be configured in the vital sign measuring module 41 except for the vital sign measuring part 42, and thus the other elements and the vital sign measuring part 42 may be formed as a single module.

The communication module 43 is disposed at an opposite side of the vital sign measuring module 41, and is spaced apart from the vital sign measuring module 41 by a predetermined distance. Various kinds of communication elements may be configured at the communication module 43.

Thus, through the communication module 43, the information obtained by the vital sign measuring module 41 and the temperature sensor 1 may be provided to outside.

A first end of the connecting part 44 is connected to the vital sign measuring module 41, and a second end of the connecting part 44 is connected to the communication module 43. Thus, the connecting part 44 connects the vital sign measuring module 41 to the communication module 43.

Here, the temperature sensor 1 is disposed inside of the connecting part 44, and the exposed portion of the measuring part 11 of the temperature sensor 1 is fixed to head for a lower portion of the connecting part 44.

Thus, the measuring part 11 is partially exposed to outside toward the lower portion of the connecting part 44, and a lower surface of the connecting part 44 makes contact with the subject body. Thus, the temperature of the subject body may be measured by the measuring part 11.

Here, the connecting part 44 has a flexible structure, and is easily bent, and thus is easily located anywhere on the subject body having various surface shapes, to measure the temperature of the subject body.

Alternatively, the temperature sensor 1 may be disposed inside of the vital sign measuring module 41 and be adjacent to the vital sign measuring part 42, not inside of the connecting part 44. Thus, the temperature sensor 1 may be prevented from being damaged due to restructuring of the flexible structure of the connecting part 44, when the temperature sensor 1 is disposed on the connecting part 44.

The first electrode 45 is attached to a lower surface of the vital sign measuring module 41, which is the surface heading for the subject body. The second electrode 46 is attached to a lower surface of the communication module 43, which is the surface heading for the subject body.

The first and second electrodes 45 and 46 are electrodes for measuring the vital signs, and thus are directly attached to the subject body. Electric signals measured by the first and second electrodes 45 and 46 may be provided to the vital sign measuring part 42 of the vital sign measuring module 41.

Thus, the vital sign measuring part 42 may obtain various kinds of vital signs of the subject body, based on the electric signals provided by the first and second electrodes 45 and 46

Here, the obtained vital signs may be the information obtained through the electric signals such as electrocardiogram signal, and other signals such as oxygen saturation, pulse, blood pressure and so on may be also obtained.

Each of the first and second electrodes 45 and 46 may be formed as a patch type attached to the subject body, and thus, the wearable device 40 according to the present example embodiment is formed as the patch type directly attached to the subject body. Then, various kinds of vital signs including the temperature may be obtained.

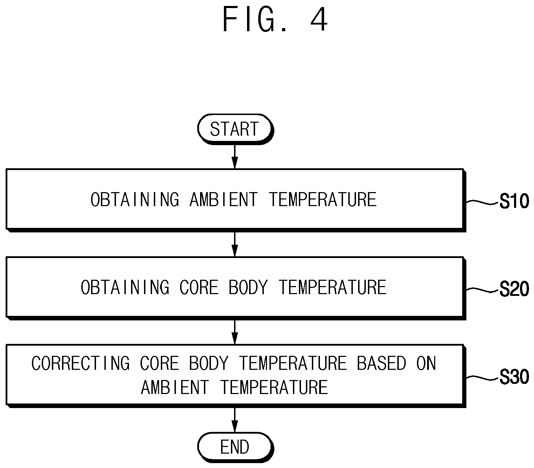

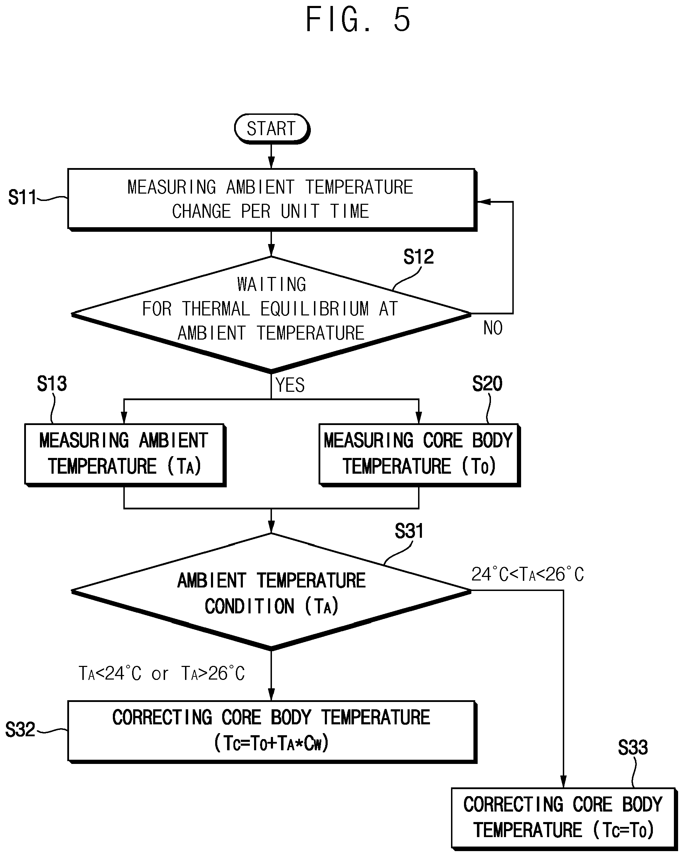

FIG. 4 is a flow chart showing a core temperature measurement method using the temperature sensor of FIG. 1A, and FIG. 5 is a flow chart showing the flow chart of FIG. 4 in detail.

Referring to FIG. 4 and FIG. 5, in measuring the core body temperature of the subject body using the temperature sensor 1 explained above referring to FIG. 1A and FIG. 1B, an ambient temperature is obtained (step S10).

Here, the temperature sensor 1 may include an additional temperature sensor configured to measure the ambient temperature, except for the measuring part 11 measuring the temperature of the subject body.

The additional temperature sensor may be a measuring sensor included by the cover part12, and then the ambient temperature may be measured.

Alternatively, the additional temperature sensor may be disposed at the substrate 20 of the temperature sensor 1, as an additional sensor. Further, the addition temperature sensor may be disposed inside of the case 35 of the band type wearable device 30 as an additional sensor, or may be disposed inside of the vital signal measuring module 41 or the connecting part 44 of the patch type wearable device 40 as an additional sensor.

In obtaining the ambient temperature (step S10), an ambient temperature change per a unit time is measured by the additional temperature sensor (step S11). Then, based on the measured result of the ambient temperature change, whether or not the ambient temperature is in thermal equilibrium is determined (step S12), and then whether or not the ambient temperature is measured is determined.

Here, whether or not the ambient temperature is in the thermal equilibrium may be determined by an additional control part (not shown), and the additional control part may determine the thermal equilibrium when the ambient temperature change is maintained within a predetermined range for a predetermined period.

Thus, when the ambient temperature is determined to be thermal equilibrium (step S12), the ambient temperature $T_A$ is measured by the additional temperature sensor (step S13).

Then, a core body temperature is obtained by the temperature sensor 1 (step S20).

Here, the temperature sensor 1 measures the temperature of the subject body using the measuring part 11 making contact with the subject body, and the measured temperature of the subject body is considered as the core body temperature $T_o$.

Then, the core body temperature is corrected based on the ambient temperature (step S30).

Here, whether or not the obtained ambient temperature $T_A$ satisfies a predetermined condition, is determined (step S31). Based on the range of the ambient temperature, the core body temperature is corrected (step S32 and step S33).

For example, when the ambient temperature $T_A$ satisfies a first temperature condition in which the temperature is in a range between 24°C and 26°C, the core body temperature is not corrected and the obtained core body temperature $T_o$ is considered as the corrected core body temperature $T_c$.

Alternatively, when the ambient temperature $T_A$ satisfies a second temperature condition in which the temperature is less than 24°C or over 26°C, the core body temperature $T_o$ is corrected using Equation (1) below.

$$T_C = T_O + T_A \times C_W \qquad \text{Equation (1)}$$

Here, $T_c$ is a corrected core body temperature, $T_o$ is a measured core body temperature, $T_A$ is a measured ambient temperature, and $C_w$ is a correcting coefficient.

The correcting coefficient $C_w$ is a predetermined coefficient as listed in an additional look-up-table. For example, the correcting coefficient may be listed as in Table 1.

The correcting coefficient $C_w$ is the predetermined coefficient based on the measured ambient temperature $T_A$, and thus, Equation (1) may be calculated based on the correcting coefficient $C_w$. Then, the corrected core body temperature $T_c$ may be obtained finally (step S32).

Accordingly, when measuring the temperature of the temperature sensor 1, the ambient temperature is firstly obtained and then the core body temperature is corrected. Thus, the core body temperature may be obtained more accurately.

[Table 1]

| Ambient Temperature $(T_A)$ [°C] | Correcting coefficient $(C_w)$ |
|---|---|
| 10.0 | -1.5 |
| 11.0 | -1.4 |

(continued)

| Ambient Temperature ($T_A$) [°C] | Correcting coefficient ($C_w$) |
|---|---|
| 12.0 | -1.3 |
| 13.0 | -1.2 |
| 14.0 | -1.1 |
| 15.0 | -1.0 |
| 16.0 | -0.9 |
| 17.0 | -0.8 |
| 18.0 | -0.7 |
| 19.0 | -0.6 |
| 20.0 | -0.5 |
| 21.0 | -0.4 |
| 22.0 | -0.3 |
| 23.0 | -0.2 |
| 24.0 | -0.1 |
| 25.0 (reference temperature) | 0.0 (no correction) |
| 26.0 | 0.1 |
| 27.0 | 0.2 |
| 28.0 | 0.3 |
| 29.0 | 0.4 |
| 30.0 | 0.5 |
| 31.0 | 0.6 |
| 32.0 | 0.7 |
| 33.0 | 0.8 |
| 34.0 | 0.9 |
| 35.0 | 1.0 |
| 36.0 | 1.1 |
| 37.0 | 1.2 |
| 38.0 | 1.3 |
| 39.0 | 1.4 |
| 40.0 | 1.5 |

According to the present embodiment, the measuring part is mounted on the substrate to be operated, but heat generated from various elements may be provided to the measuring part through the substrate, so that the measured temperature of the subject body may be inaccurate.

Thus, in the present example embodiments, the plurality of slits is formed in the substrate and thus a first area in which the sensing unit is formed is isolated. Thus, the heat transfer through the substrate may be minimized and the measured temperature of the subject body may be accurate.

Here, a portion of the measuring part heading for the subject body is merely exposed and other entire of the measuring part is blocked by the cover, and thus measured temperature of the subject body may be accurate.

In addition, the temperature sensor may be applied to the band type or the patch type wearable device. When applied to the patch type wearable device, the temperature sensor may be mounted on the connecting part having the flexible structure, to increase the adhesiveness with the subject body.

Further, in the wearable device, various kinds of vital signs in addition to the body temperature may be obtained, and the vital signs may be obtained using the electrode making contact with the subject body.

Here, in measuring the core body temperature, to increase the accuracy of the measurement, the core body temperature is corrected using the ambient temperature.

Having described exemplary embodiments of the present invention, it is further noted that it is readily apparent to those of reasonable skill in the art that various modifications may be made without departing from the spirit and scope of the invention which is defined by the metes and bounds of the appended claims.

**Claims**

1. A temperature sensor comprising:

   a sensing unit comprising a base part, a measuring part mounted on the base part and configured to measure a temperature of a subject body by making contact with the subject body, and a cover part configured to cover the measuring part; and
   a substrate on which the sensing unit is mounted,
   wherein a plurality of slits is formed around a first area of the substrate, and the sensing unit is mounted at the first area of the substrate.

2. The temperature sensor of claim 1, wherein the sensing unit further comprises:
   a terminal part extending from the base part toward an opposite to the measuring part, and passing through the substrate to be electrically connected to the substrate.

3. The temperature sensor of claim 1, wherein the cover part covers an entire of the measuring part except for a portion of the measuring part heading for the subject body, and the portion of the measuring part exposed toward the subject body makes contact with the subject body for measuring the temperature of the subject body.

4. The temperature sensor of claim 3, wherein the cover part comprises:
   a measuring sensor configured to measure an ambient temperature.

5. The temperature sensor of claim 1, wherein the slits are spaced apart from the first area by a predetermined distance, and the slits are spaced apart from each other by a predetermined distance.

6. The temperature sensor of claim 5, wherein the distance between the slits adjacent to each other, is smaller than a length of each of the slits.

7. A wearable device comprising:

   the temperature sensor of claim 1;
   a vital sign measuring part configured to measure vital signs of the subject body except for the temperature of the subject body;
   a display part configured to display results of the temperature sensor and the vital sign measuring part; and
   a case receiving the temperature sensor, the vital sign measuring part and the display part.

8. The wearable device of claim 7, wherein the measuring part of the temperature sensor is exposed to an outside of the case to make contact with the subject body.

9. A wearable device comprising:

   the temperature sensor of claim 1;
   a vital sign measuring module comprising a vital sign measuring part, wherein the vital sign measuring part is configured to measure vital signs of the subject body except for the temperature of the subject body;
   a communication module disposed to an opposite to the vital sign measuring module and is configured to communicate with outside; and
   a connecting part configured to connect the vital sign measuring module with the communication module,
   wherein the temperature sensor is disposed inside of the vital sign measuring module or the connecting part.

10. The wearable device of claim 9, wherein the connecting part is a flexible structure, and the measuring part of the temperature sensor is exposed to an outside of the connecting part or an outside of the vital sign measuring module to make contact with the subject body.

**11.** The wearable device of claim 9, further comprising:

a first electrode attached to a lower portion of the vital sign measuring module to make contact with the subject body; and
a second electrode attached to a lower portion of the communication part to make contact with the subject body.

**12.** A core temperature measurement method using the temperature sensor of claim 1, the method comprising:

obtaining an ambient temperature;
obtaining a core body temperature using the temperature sensor; and
correcting the core body temperature based on the ambient temperature.

**13.** The method of claim 12, wherein in the correcting the core body temperature,

the ambient temperature is compared to the core body temperature, and then when the ambient temperature satisfies a first temperature condition, the core body temperature is not corrected and is considered to be a core temperature of the subject body, and
when the ambient temperature satisfies a second temperature condition, the core body temperature is corrected to obtain a core temperature of the subject body.

**14.** The method of claim 13, wherein in the first temperature condition, the ambient temperature is in a range between 24°C and 26°C, and in the second temperature condition, the ambient temperature is less than 24°C or over 26°C.

**15.** The method of claim 13, wherein in the correcting the core body temperature, the core body temperature is corrected using Equation (1) below,

$$T_C = T_O + T_A \times C_W \qquad \text{Equation (1)}$$

wherein, $T_c$ is a corrected core body temperature, $T_o$ is a measured core body temperature, $T_A$ is a measured ambient temperature, and $C_w$ is a correcting coefficient.

# FIG. 1A

# FIG. 1B

# FIG. 2A

# FIG. 2B

FIG. 2C

# FIG. 3A

# FIG. 3B

# FIG. 4

START

OBTAINING AMBIENT TEMPERATURE ～S10

OBTAINING CORE BODY TEMPERATURE ～S20

CORRECTING CORE BODY TEMPERATURE BASED ON AMBIENT TEMPERATURE ～S30

END

# FIG. 5

START

S11 — MEASURING AMBIENT TEMPERATURE CHANGE PER UNIT TIME

S12 — WAITING FOR THERMAL EQUILIBRIUM AT AMBIENT TEMPERATURE

NO

YES

S13 — MEASURING AMBIENT TEMPERATURE ($T_A$)

S20 — MEASURING CORE BODY TEMPERATURE ($T_0$)

S31 — AMBIENT TEMPERATURE CONDITION ($T_A$)

$24°C < T_A < 26°C$

$T_A < 24°C$ or $T_A > 26°C$

S32 — CORRECTING CORE BODY TEMPERATURE ($T_C = T_0 + T_A * C_W$)

S33 — CORRECTING CORE BODY TEMPERATURE ($T_C = T_0$)

## INTERNATIONAL SEARCH REPORT

| International application No. |
| --- |
| **PCT/KR2021/012978** |

**A.    CLASSIFICATION OF SUBJECT MATTER**

**A61B 5/01**(2006.01)i; **A61B 5/00**(2006.01)i

According to International Patent Classification (IPC) or to both national classification and IPC

**B.    FIELDS SEARCHED**

Minimum documentation searched (classification system followed by classification symbols)

A61B 5/01(2006.01); A61B 5/00(2006.01); A61B 5/02(2006.01); A61B 5/0205(2006.01); G01K 1/16(2006.01); G06Q 50/02(2012.01); H05K 7/20(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심부 체온(core temperature), 센서(sensor), 웨어러블(wearable), 표시(display), 통신(communication)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2016-206024 A (FUJITSU PERIPHERALS LTD.) 08 December 2016 (2016-12-08)<br>     See paragraphs [0002]-[0027]; and figure 5. | 1,3-6 |
| Y | | 2,7-15 |
| Y | JP 2018-518323 A (RHYTHM DIAGNOSTIC SYSTEMS, INC.) 12 July 2018 (2018-07-12)<br>     See paragraphs [0027] and [0029]; and figure 1A. | 2,9-11 |
| Y | CN 211723138 U (ZHOU, Zhongquan) 23 October 2020 (2020-10-23)<br>     See paragraph [0015]. | 7,8 |
| Y | KR 10-1195115 B1 (SMEC CO., LTD.) 29 October 2012 (2012-10-29)<br>     See paragraphs [0028]-[0057]. | 12-15 |
| A | CN 102281816 A (BODYMEDIA, INC. et al.) 14 December 2011 (2011-12-14)<br>     See entire document. | 1-15 |

☐ Further documents are listed in the continuation of Box C.          ☑ See patent family annex.

| * | Special categories of cited documents: | | "T" | later document published after the international filing date or priority date and not in conflict with the application but cited to understand the principle or theory underlying the invention |
| --- | --- | --- | --- | --- |
| "A" | document defining the general state of the art which is not considered to be of particular relevance | | | |
| "D" | document cited by the applicant in the international application | | "X" | document of particular relevance; the claimed invention cannot be considered novel or cannot be considered to involve an inventive step when the document is taken alone |
| "E" | earlier application or patent but published on or after the international filing date | | | |
| "L" | document which may throw doubts on priority claim(s) or which is cited to establish the publication date of another citation or other special reason (as specified) | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | | |
| "P" | document published prior to the international filing date but later than the priority date claimed | | "&" | document member of the same patent family |

| Date of the actual completion of the international search | Date of mailing of the international search report |
| --- | --- |
| **03 March 2022** | **03 March 2022** |

| Name and mailing address of the ISA/KR | Authorized officer |
| --- | --- |
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

| International application No. |
|---|
| **PCT/KR2021/012978** |

| Patent document cited in search report | | | Publication date (day/month/year) | Patent family member(s) | | | Publication date (day/month/year) |
|---|---|---|---|---|---|---|---|
| JP | 2016-206024 | A | 08 December 2016 | None | | | |
| JP | 2018-518323 | A | 12 July 2018 | CA | 2886858 | A1 | 10 April 2014 |
| | | | | CA | 2935598 | A1 | 30 July 2015 |
| | | | | CA | 2935598 | C | 26 October 2021 |
| | | | | CA | 2983771 | A1 | 29 December 2016 |
| | | | | CA | 3045025 | A1 | 21 June 2018 |
| | | | | CN | 104812296 | A | 29 July 2015 |
| | | | | CN | 104812296 | B | 01 May 2018 |
| | | | | CN | 105960197 | A | 21 September 2016 |
| | | | | CN | 107735025 | A | 23 February 2018 |
| | | | | CN | 110087543 | A | 02 August 2019 |
| | | | | EP | 2903509 | A1 | 12 August 2015 |
| | | | | EP | 2903509 | B1 | 04 September 2019 |
| | | | | EP | 3099224 | A1 | 07 December 2016 |
| | | | | EP | 3099224 | B1 | 20 May 2020 |
| | | | | EP | 3313287 | A1 | 02 May 2018 |
| | | | | EP | 3554366 | A1 | 23 October 2019 |
| | | | | EP | 3554366 | A4 | 02 September 2020 |
| | | | | EP | 3636148 | A2 | 15 April 2020 |
| | | | | EP | 3636148 | A3 | 20 May 2020 |
| | | | | EP | 3769669 | A1 | 27 January 2021 |
| | | | | ES | 2824126 | T3 | 11 May 2021 |
| | | | | FR | 3015100 | A1 | 19 June 2015 |
| | | | | FR | 3015100 | B1 | 25 December 2015 |
| | | | | JP | 2015-530225 | A | 15 October 2015 |
| | | | | JP | 2017-510390 | A | 13 April 2017 |
| | | | | JP | 2018-126518 | A | 16 August 2018 |
| | | | | JP | 2019-141704 | A | 29 August 2019 |
| | | | | JP | 2020-513876 | A | 21 May 2020 |
| | | | | JP | 2021-053464 | A | 08 April 2021 |
| | | | | JP | 2021-180876 | A | 25 November 2021 |
| | | | | JP | 6298063 | B2 | 20 March 2018 |
| | | | | JP | 6539827 | B2 | 10 July 2019 |
| | | | | JP | 6625682 | B2 | 25 December 2019 |
| | | | | JP | 6826735 | B2 | 10 February 2021 |
| | | | | US | 10080527 | B2 | 25 September 2018 |
| | | | | US | 10244949 | B2 | 02 April 2019 |
| | | | | US | 10413251 | B2 | 17 September 2019 |
| | | | | US | 10610159 | B2 | 07 April 2020 |
| | | | | US | 10842391 | B2 | 24 November 2020 |
| | | | | US | 10863947 | B2 | 15 December 2020 |
| | | | | US | 10959678 | B2 | 30 March 2021 |
| | | | | US | 10980486 | B2 | 20 April 2021 |
| | | | | US | 10993671 | B2 | 04 May 2021 |
| | | | | US | 10D850626 | S | 04 June 2019 |
| | | | | US | 2014-0100432 | A1 | 10 April 2014 |
| | | | | US | 2014-0189881 | A1 | 03 July 2014 |
| | | | | US | 2015-0094552 | A1 | 02 April 2015 |
| | | | | US | 2015-0096050 | A1 | 02 April 2015 |
| | | | | US | 2015-0148622 | A1 | 28 May 2015 |

Form PCT/ISA/210 (patent family annex) (July 2019)

**INTERNATIONAL SEARCH REPORT**
Information on patent family members

International application No.

**PCT/KR2021/012978**

| Patent document cited in search report | | | Publication date (day/month/year) | Patent family member(s) | | | Publication date (day/month/year) |
|---|---|---|---|---|---|---|---|
| | | | | US | 2015-0148637 | A1 | 28 May 2015 |
| | | | | US | 2015-0148691 | A1 | 28 May 2015 |
| | | | | US | 2015-0170527 | A1 | 18 June 2015 |
| | | | | US | 2016-0302674 | A1 | 20 October 2016 |
| | | | | US | 2018-0028122 | A1 | 01 February 2018 |
| | | | | US | 2018-0177459 | A1 | 28 June 2018 |
| | | | | US | 2019-0029599 | A1 | 31 January 2019 |
| | | | | US | 2019-0320914 | A1 | 24 October 2019 |
| | | | | US | 2020-0008749 | A1 | 09 January 2020 |
| | | | | US | 2020-0229767 | A1 | 23 July 2020 |
| | | | | US | 2021-0100514 | A1 | 08 April 2021 |
| | | | | US | 2021-0145293 | A1 | 20 May 2021 |
| | | | | US | 8935800 | B2 | 13 January 2015 |
| | | | | US | 9582434 | B2 | 28 February 2017 |
| | | | | US | 9773423 | B2 | 26 September 2017 |
| | | | | US | 9782132 | B2 | 10 October 2017 |
| | | | | WO | 2014-055994 | A1 | 10 April 2014 |
| | | | | WO | 2015-113054 | A1 | 30 July 2015 |
| | | | | WO | 2016-210334 | A1 | 29 December 2016 |
| | | | | WO | 2018-112401 | A1 | 21 June 2018 |
| CN | 211723138 | U | 23 October 2020 | None | | | |
| KR | 10-1195115 | B1 | 29 October 2012 | None | | | |
| CN | 102281816 | A | 14 December 2011 | AU | 2009-318098 | A1 | 27 May 2010 |
| | | | | AU | 2009-318098 | B2 | 16 July 2015 |
| | | | | CA | 2744016 | A1 | 27 May 2010 |
| | | | | CA | 2744016 | C | 11 December 2018 |
| | | | | CA | 2744215 | A1 | 10 June 2010 |
| | | | | CA | 2747057 | A1 | 08 July 2010 |
| | | | | CN | 102281816 | B | 07 January 2015 |
| | | | | EP | 2358266 | A1 | 24 August 2011 |
| | | | | EP | 2367798 | A2 | 28 September 2011 |
| | | | | EP | 2367798 | B1 | 28 February 2018 |
| | | | | EP | 2375973 | A2 | 19 October 2011 |
| | | | | HK | 1164675 | A1 | 28 September 2012 |
| | | | | IL | 212992 | A | 31 July 2011 |
| | | | | IL | 213004 | A | 31 July 2011 |
| | | | | IL | 213004 | B | 30 April 2014 |
| | | | | IL | 231968 | A | 28 May 2014 |
| | | | | US | 10105366 | B2 | 23 October 2018 |
| | | | | US | 10167263 | B2 | 01 January 2019 |
| | | | | US | 2011-0237907 | A1 | 29 September 2011 |
| | | | | US | 2012-0123232 | A1 | 17 May 2012 |
| | | | | US | 2012-0196883 | A1 | 02 August 2012 |
| | | | | US | 2012-0245439 | A1 | 27 September 2012 |
| | | | | US | 2015-0018388 | A1 | 15 January 2015 |
| | | | | US | 2016-0016911 | A1 | 21 January 2016 |
| | | | | US | 2016-0310491 | A1 | 27 October 2016 |
| | | | | US | 9145424 | B2 | 29 September 2015 |
| | | | | US | 9428467 | B2 | 30 August 2016 |
| | | | | US | 9809556 | B2 | 07 November 2017 |

Form PCT/ISA/210 (patent family annex) (July 2019)

EP 4 265 176 A1

**INTERNATIONAL SEARCH REPORT**
Information on patent family members

International application No.

**PCT/KR2021/012978**

| Patent document cited in search report | Publication date (day/month/year) | Patent family member(s) | | | Publication date (day/month/year) |
|---|---|---|---|---|---|
| | | WO | 2010-059241 | A2 | 27 May 2010 |
| | | WO | 2010-059241 | A3 | 16 September 2010 |
| | | WO | 2010-065067 | A1 | 10 June 2010 |
| | | WO | 2010-077997 | A2 | 08 July 2010 |
| | | WO | 2010-077997 | A3 | 14 October 2010 |
| | | WO | 2012-100073 | A1 | 26 July 2012 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200061579

- KR 1020200061580